# EUROPEAN PATENT APPLICATION

(11) **EP 4 847 043 A1**
(43) Date of publication of application: **02.12.2026**
(21) Application number: 25749204.1
(22) Date of filing: 31.01.2025
(51) Int. Cl.: A61K 41/00, A61K 9/51, A61K 47/69, A61K 51/12, A61K 51/08, A61K 49/00, A61P 35/00

(54) **ALBUMIN NANOPLATFORM FOR BORON NEUTRON CAPTURE THERAPY AND COMPOSITION FOR BORON NEUTRON CAPTURE THERAPY COMPRISING SAME**

(30) Priority: 31.01.2024 KR 20240014785
(71) Applicant: Clichembio, Inc., Seoul 03122 (KR)
(72) Inventor: LEE, Yun Sang, Yongin-si, Gyeonggi-do 16851 (KR); PARK, Ji Yong, Incheon 22002 (KR); YOO, Ran Ji, Seoul 01727 (KR); KIM, Kyuwan, Seoul 02264 (KR); NIWA, Takashi, Fukuoka-si, Fukuoka 812--0034 (JP); HOSOYA, Takamitsu, Tokyo 136-0072 (JP)
(74) Representative: AWA Sweden AB
(86) International application number: PCT/KR2025/001605
(87) International publication number: WO 2025/165163

(57) **Abstract**

The present invention relates to an albumin nanoplatform for boron neutron capture therapy and a composition for boron neutron capture therapy comprising the same. The albumin-based nanoplatform according to the present invention can effectively deliver boron to a specific cancer tissue by conjugating, as transmitters using a click chemistry functional group introduced onto the surface of albumin, a boron compound comprising an excessive amount of boron together with a targeting molecule that specifically targets a cancer tissue. In particular, since it can deliver a sufficient amount of boron molecules to a target cancer tissue even with a dose that is more than 10 times smaller than that of a conventional boron neutron capture therapy (BNCT) drug, it can be utilized as a composition for boron neutron capture therapy and as an anticancer therapeutic agent.

## Description

### [Technical Field]

The present invention relates to an albumin nanoplatform for boron neutron capture therapy and a composition for boron neutron capture therapy comprising the same, and more particularly, to a technique for providing an albumin-based composition for boron neutron capture therapy that selectively targets a cancer tissue for boron neutron capture therapy and can improve an anticancer therapeutic effect by stably and effectively delivering boron to the cancer tissue even with a small dose.

### [Background Art]

Neutron capture therapy (NCT) is a therapeutic technique that selectively destroys only tumor cells by accumulating a neutron capture drug at a tumor site and then irradiating the site with thermal neutrons. Since low-energy thermal neutrons cause a nuclear fission reaction selectively only in cancer cells in which the drug has accumulated while causing little damage to normal cells, neutron capture therapy is a technique that is very effective for cancer treatment compared to conventional radiation therapy. Therefore, a strategy of intensively accumulating a drug only in tumor cells in order to prevent damage to normal tissues, which is a major problem of radiation therapy, is at the core of neutron capture therapy technology.

Boron neutron capture therapy (BNCT), which uses boron as a neutron capture drug, is based on the principle of a ¹⁰B(n,α)⁷Li nuclear fission reaction. A ¹⁰B atom, which is an isotope of boron, captures a thermal neutron to cause a nuclear fission reaction and decays to generate alpha and lithium particles and gamma rays. Here, the range of the emitted alpha and lithium particles is about 5 to 7 micrometers, which corresponds to the diameter of a cell having a size of about 10 micrometers, so that damage is ultimately caused only to cells adjacent to the boron drug. Therefore, BNCT has been expected as a therapy capable of selectively treating only tumor cells while minimizing damage to normal cells by using a boron drug that selectively accumulates in tumor cells.

Such boron neutron capture therapy has been used for the treatment and research of various cancer types such as brain tumors, head and neck cancer, melanoma, and colorectal cancer, but has not been widely commercialized due to the problem of the accumulation rate of boron compounds in cancer cells and the limitation of requiring a nuclear reactor facility.

Representative boron drugs currently used in clinical practice are BPA (p-boronophenylalanine) and BSH (sulfhydryl borane, Na₂B₁₂H₁₁SH). BPA is an amino acid derivative and is known to accumulate well in cancer tissues in which protein synthesis is active; however, due to the nature of amino acids, it is rapidly excreted through the kidneys in the body, and thus it must be administered over about 2 hours to perform BNCT. In addition, since BSH is structurally very hydrophobic, when administered into the body, most of it is initially taken up by the liver and excreted into the intestine, so that there is a problem in that the drug must be administered in an excessive amount to obtain a desired therapeutic effect.

Korean Patent Publication No. 10-2008-0107309 is known as a technique for maintaining a high intracellular boron concentration by providing a method for preparing a nanohybrid in which a boron compound is introduced into a layer of a metal double-layered hydroxide, and the nanohybrid prepared by the method, as a composition for boron neutron capture therapy; however, it only discloses a nanohybrid using metal ions and does not disclose a nanocarrier capable of delivering a sufficient amount of boron molecules to a target cancer tissue with administration of a smaller dose than conventional boron neutron capture therapy (BNCT) drugs by using a biologically derived material.

Therefore, there is a continuous need for research on the development of a satisfactory radiopharmaceutical for neutron capture therapy that can solve the problem of administering an excessive amount of a boron compound while exhibiting high biocompatibility and stability.

### [Disclosure]

### [Technical Problem]

An object of the present invention is to provide an albumin-based nanoplatform for boron neutron capture therapy (BNCT).

Another object of the present invention is to provide a composition for boron neutron capture therapy comprising the albumin-based nanoplatform.

Still another object of the present invention is to provide a composition for preventing or treating a cancer disease, comprising the albumin-based nanoplatform for boron neutron capture therapy as an active ingredient.

### [Technical Solution]

The present invention provides an albumin-based nanoplatform for boron neutron capture therapy (BNCT), obtained by a click chemistry reaction between albumin conjugated with an azide (N₃) or cyclooctyne functional group and a transmitter conjugated with an azide (N₃) or cyclooctyne functional group, wherein the transmitter comprises at least one selected from the group consisting of a boron compound and a targeting molecule, and when the albumin is conjugated with the azide functional group, the transmitter is conjugated with the cyclooctyne functional group, and when the albumin is conjugated with the cyclooctyne functional group, the transmitter is conjugated with the azide functional group.

In the present invention, the azide or cyclooctyne functional group introduced into the albumin may range from 1 to 30.

In the present invention, the nanoplatform may comprise 1 to 15 boron compounds.

In the present invention, the boron compound may be at least one selected from the group consisting of carborane and sodium borocaptate (BSH).

In the present invention, the nanoplatform may comprise 1 to 10 targeting molecules.

In the present invention, the targeting molecule may comprise at least one selected from the group consisting of folate or a derivative thereof, a saccharide compound, and RGD (arginyl-glycyl-aspartic acid) or a derivative thereof.

In the present invention, the transmitter may further comprise a radioactive isotope, and the radioactive isotope may be at least one selected from the group consisting of ³H, ¹¹C, ¹⁸F, ¹⁴Cl, ³²P, ³⁵S, ³⁶Cl, ⁴⁵Ca, ⁵¹Cr, ⁵⁷Co, ⁵⁸Co, ⁵⁹F, ⁶⁴Cu, ⁶⁷Ga, ⁶⁸Ga, ⁸⁹Zr, ⁹⁰Y, ⁹⁹Mo, ^{99m}Tc, ¹¹¹In, ¹³¹I, ¹²⁵I, ¹²⁴I, ¹²³I, ¹⁸⁶Re, ¹⁸⁸Re, ²²⁵Ac, ²¹²Pb, ^{117m}Sn, and ¹⁷⁷Lu.

In the present invention, the radioactive isotope may be labeled by a chelating agent, and the chelating agent may be at least one selected from the group consisting of NOTA (1,4,7-triazacyclononane-1,4,7-triacetic acid), DOTA (1,4,7,10-tetraazacyclododecane-1,4,7,10-tetraacetic acid), DFO (3-[6,17-dihydroxy-7,10,18,21-tetraoxo-27-[N-acetylhydroxylamino]-6,11,17,22-tetraazaheptaeicosane]thiourea), DTPA (diethylenetriaminepentaacetic acid), N₂S₂ (diaminedithiol), p-SCN-Bn-NOTA (2-(4'-isothiocyanatobenzyl)-1,4,7-triazacyclononane-1,4,7-triacetic acid), NODAGA (1,4,7-triazacyclononane-1-glutaric acid-4,7-acetic acid), p-SCN-Bn-DOTA (2-(4'-isothiocyanatobenzyl)-1,4,7,10-tetraazacyclododecane-1,4,7,10-tetraacetic acid), TETA (1,4,8,11-tetraazacyclotetradecane-1,4,8,11-tetraacetic acid), p-SCN-Bn-DTPA (2-(4-isothiocyanatobenzyl)-diethylenetriaminepentaacetic acid), p-SCN-Bn-DFO (1-(4-isothiocyanatophenyl)-3-[6,17-dihydroxy-7,10,18,21-tetraoxo-27-[N-acetylhydroxylamino]-6,11,17,22-tetraazaheptaeicosane]thiourea), and HYNIC (hydrazinonicotinic acid).

In the present invention, the transmitter may further comprise a fluorescent material, and the fluorescent material may be at least one selected from the group consisting of FNR (Ferredoxin NADP(+) reductase), a cyanine-based fluorescent material, TAMRA (tetramethylrhodamine-5-maleimide), Flamma^{®} fluorescent material, and ICG (indocyanine green).

In the present invention, the nanoplatform may selectively target a cancer cell or cancer tissue in which a folate receptor is overexpressed.

The present invention also provides a composition for boron neutron capture therapy, comprising the albumin-based nanoplatform for boron neutron capture therapy.

The present invention also provides a pharmaceutical composition for preventing or treating a cancer disease, comprising the albumin-based nanoplatform for boron neutron capture therapy.

In the present invention, the cancer disease may comprise a cancer cell or cancer tissue in which a folate receptor is overexpressed.

In the present invention, the cancer disease may be selected from head and neck cancer, ovarian cancer, lung cancer, cervical cancer, bone cancer, breast cancer, central or peripheral nervous system cancer, gastrointestinal cancer, germ cell cancer, adenocarcinoma, blood cancer, renal-urinary tract cancer, liver cancer, pleural cancer, prostate cancer, sarcoma, and skin cancer.

### [Advantageous Effects]

The albumin-based nanoplatform according to the present invention can effectively deliver boron to a specific cancer tissue by conjugating, as transmitters using a click chemistry functional group introduced onto the surface of albumin, a boron compound comprising an excessive amount of boron together with a targeting molecule that specifically targets a cancer tissue, in particular, the albumin-based nanoplatform of the present invention can deliver a sufficient amount of boron molecules to a target cancer tissue even with a dose that is more than 10 times smaller than that of conventional boron neutron capture therapy (BNCT) drugs, and thus can be utilized as a composition for boron neutron capture therapy and as an anticancer therapeutic agent.

### [Description of Drawings]

FIG. 1 is a schematic diagram of a process for identifying pharmacokinetic properties of a drug in a mouse model using a drug labeled with a radioactive isotope, according to an embodiment of the present invention.
FIG. 2 is a schematic diagram of an albumin-based nanoplatform for boron neutron capture therapy that targets cancer, according to an embodiment of the present invention.
FIG. 3 shows a reaction scheme for synthesizing azide-conjugated carborane according to an embodiment of the present invention.
FIGS. 4a and 4b are fluorescence analysis results confirming the cancer cell targeting and internalization ability of an albumin nanoplatform according to the number of conjugated folates, according to an embodiment of the present invention.
FIG. 5 is a fluorescence analysis result confirming the cancer cell targeting ability of an albumin nanoparticle conjugated with folate and [¹⁰B]carborane (FA-ANP-FNR648) and an albumin nanoparticle not conjugated with folate (ANP-FNR648), with respect to a head and neck cancer cell line in which a folate receptor is overexpressed, according to an embodiment of the present invention.
FIG. 6a is a fluorescence analysis result confirming the targeting ability of each nanoparticle to a cancer tissue after injecting Alb-ADIBO¹¹-[¹⁰B]⁶-FA⁵-FNR648 or Alb-ADIBO¹¹-FA³-[¹⁰B]⁶-FNR648 into a cancer mouse model constructed using a head and neck cancer cell line, according to an embodiment of the present invention.
FIG. 6b compares the albumin nanoplatform uptake amounts of a KB cell line and a FaDu cell line, according to an embodiment of the present invention.
FIG. 7 is a standard graph of intensity with respect to the concentration of boron used when an albumin nanoplatform is labeled with a fluorescent material, according to an embodiment of the present invention.
FIG. 8a shows a result of confirming, by Radio-TLC, the efficiency with which a radioactive isotope is labeled on an albumin nanoplatform, according to an embodiment of the present invention.
FIGS. 8b and 8c are PET imaging results confirming the targeting ability of each nanoparticle to a tumor tissue after injecting ⁶⁴Cu-NOTA-Alb-ADIBO¹¹-B¹⁰-FA or ⁶⁴Cu-NOTA-Alb-ADIBO¹¹-FA-B¹⁰ into a cancer mouse model constructed using a head and neck cancer cell line, according to an embodiment of the present invention.
FIG. 9 is a standard graph of intensity with respect to the concentration of boron used when an albumin nanoplatform is labeled with a radioactive isotope, according to an embodiment of the present invention.
FIG. 10 is a graph of the correlation between a PET signal and a boron concentration, according to an embodiment of the present invention.

### [Best Mode for Invention]

Hereinafter, specific embodiments of the present invention will be described in more detail. Unless otherwise defined, all technical and scientific terms used herein have the same meanings as commonly understood by those skilled in the art to which the present invention pertains. In general, the nomenclature used herein is well-known and commonly used in the art.

The present invention relates to an albumin-based nanoplatform for boron neutron capture therapy for selectively targeting a cancer tissue and delivering boron into the cancer tissue for boron neutron capture therapy (BNCT), a composition for boron neutron capture therapy comprising the same, and a composition for preventing or treating a cancer disease.

Target-directed drug delivery platforms have been diversified using convergent new materials along with technological development. However, the utilization of such drug delivery platforms is limited due to the injection time in the body or rapid uptake by the spleen. In particular, problems regarding an increase in the amount used and immune responses have been raised, and the inherent toxicity of conventional nanoparticles depending on their material composition has become a problem. In the present invention, it was confirmed that an albumin-based nanocarrier, in which an excessive amount of a boron compound is conjugated to a single albumin particle using albumin, which is a biologically derived material having high affinity in the body, effectively targets a cancer tissue and delivers a high concentration of a boron compound with administration of only a small amount, and further, it was discovered that the albumin-based nanocarrier itself can be used to treat a cancer disease.

More specifically, one aspect of the present invention provides an albumin-based nanoplatform for boron neutron capture therapy, obtained by a click chemistry reaction between albumin conjugated with an azide (N₃) or cyclooctyne functional group and a transmitter conjugated with an azide (N₃) or cyclooctyne functional group,
wherein the transmitter comprises at least one selected from the group consisting of a boron compound and a targeting molecule, and
when the albumin is conjugated with the azide functional group, the transmitter is conjugated with the cyclooctyne functional group, and when the albumin is conjugated with the cyclooctyne functional group, the transmitter is conjugated with the azide functional group.

In the present invention, albumin refers to a protein that constitutes the basic materials of cells, is abundantly present in the blood, and is produced in the liver. The albumin has the lowest molecular weight among simple proteins that exist in nature. Serum albumin in the blood functions to maintain and restore plasma volume, thereby preventing shock caused by excessive bleeding, and is used in surgery and burn treatments. It is also known to possess an oxygen transport capability similar to that of hemoglobin.

The albumin may include any albumin capable of being formulated, but may preferably be derived from human plasma or may be, but is not limited to, recombinant human serum albumin produced by genetic engineering. Genetic information regarding albumin in the present invention may be obtained from known databases, such as NCBI GenBank.

In the present invention, the number of amino groups (-NH₂) exposed on the surface of the albumin may range from 15 to 30.

In a specific embodiment of the present invention, human serum albumin (HSA), a representative biocompatible albumin, was used to prepare a nanoplatform capable of exhibiting a selective anticancer therapeutic effect on a target cancer tissue by targeting a cancer tissue and effectively delivering a boron compound based on biocompatible albumin, and a cyclooctyne or azide (N₃) group, which is one functional group for click chemistry, was introduced onto the surface of the human serum albumin under reaction conditions that minimized denaturation of the HSA.

In the present invention, azide (N₃) is a reactive group composed of three nitrogen atoms and has high reactivity. In particular, it is known to act as an electron donor in a 1,3-dipolar cycloaddition reaction, which is a type of Cu-Free click chemistry, and to play a role in forming a triaza-5-membered ring.

The cyclooctyne functional group is an 8-membered aliphatic ring comprising a triple bond that undergoes ring strain. In particular, it is known to act as an electron acceptor in a 1,3-dipolar cycloaddition reaction, which is a type of Cu-Free click chemistry, and to play a role in forming a triaza-5-membered ring. Due to the structural characteristics of the cyclooctyne, that is, the triple bond structure under ring strain, click chemistry is possible without a Cu(I) catalyst.

The cyclooctyne functional group may be, but is not necessarily limited to, at least one selected from the group consisting of 4-cyclooctyn-1-yl ( 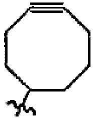 ), 3-cyclooctyn-1-yl ( 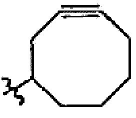 ), 2-cyclooctyn-1-yl ( 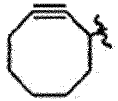 ), Monofluorinated cyclooctyne (MOFO) ( 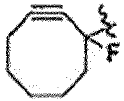 ), Difluororinated cyclooctyne (DIFO) ( 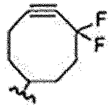 ), Dimethoxyazacyclooctyne (DIMAC) ( 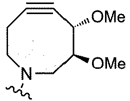 ), Dibenzocyclooctyne (DIBO) ( 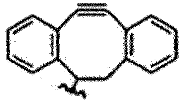 ), Azadibenzocyclooctyne (ADIBO) ( 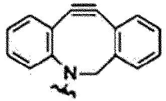 ), and biarylazacyclooctynone (BARAC) ( 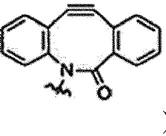 ).

Albumin conjugated with the azide (N₃) or cyclooctyne functional group may be obtained by the following steps: (a) dissolving albumin in a phosphate-buffered saline (PBS), (b) dissolving azide-NHS or cyclooctyne-NHS in DMSO, and (c) mixing the obtained solutions and then reacting them at 20 to 37°C for 30 minutes to 1 hour.

In step (a), the phosphate-buffered saline (PBS) may have a pH of 6.8 to 7.6, and preferably a pH of 7.0 to 7.4.

In step (b), the amount of DMSO used to prepare the azide-NHS solution or cyclooctyne-NHS solution may be 2% (v/v) or less of the total reaction solution.

In step (c), the molar mixing ratio of albumin to azide-NHS or cyclooctyne-NHS may range from 1:1 to 1:25.

In step (c), when the albumin solution is mixed with the azide-NHS solution, the functional group conjugated to the albumin may be the azide functional group, and when the albumin solution is mixed with the cyclooctyne-NHS solution, the functional group conjugated to the albumin may be the cyclooctyne functional group.

In one embodiment of the present invention, a human serum albumin (HSA) solution was mixed with an ADIBO-NHS solution and reacted at 37°C for 30 minutes to prepare HSA-ADIBO.

The number of click reaction functional groups (azide or cyclooctyne functional groups) introduced onto the surface of the albumin is preferably 1 to 30, and more preferably 6 to 20. In this case, upon injection into the human body, the functional groups can remain in the blood for a long time and increase the possibility of uptake at the target site. In contrast, if the number of click reaction functional groups exceeds the above range, they may be immediately taken up by the liver upon injection into the body, thereby limiting uptake at other target disease sites.

The number of click reaction functional groups introduced onto the surface of the albumin may be adjusted according to the reaction ratio of the albumin to the azide-NHS or cyclooctyne-NHS.

The albumin-based nanoplatform for boron neutron capture therapy, which is one aspect of the present invention, is obtained by mixing a solution comprising the aforementioned albumin conjugated with the azide (N₃) or cyclooctyne functional group and a solution comprising a transmitter conjugated with the azide (N₃) or cyclooctyne functional group and performing a click chemistry reaction, wherein the click chemistry reaction may be a copper catalyst-free (Cu-free) click chemistry reaction.

In one aspect of the present invention, since the azide functional group used as the click chemistry functional group is an electron donor and the cyclooctyne functional group is an electron acceptor, when the functional group conjugated to the albumin is the azide functional group, the functional group conjugated to the transmitter is preferably the cyclooctyne functional group, and when the functional group conjugated to the albumin is the cyclooctyne functional group, the functional group conjugated to the transmitter is preferably the azide functional group.

Since the albumin-based nanoplatform for boron neutron capture therapy of the present invention has a plurality of click reaction functional groups, various transmitters can be conjugated to a plurality of reactive groups.

The transmitter refers to a substance that is conjugated to the albumin and delivered into the body, and in the present invention, the transmitter comprises at least one of a boron compound and a targeting molecule.

The boron compound is conjugated to the albumin nanoplatform of the present invention and delivered to a target cancer cell so as to enable effective boron neutron capture therapy.

As the boron compound used in the present invention, carborane, sodium borocaptate (BSH), or the like may be used, and carborane is preferable in that it can conjugate a larger amount of boron to the albumin. In an embodiment of the present invention, it was confirmed that a larger amount of boron can be delivered to cancer cells with a small dose by using [¹⁰B]carborane comprising 10 borons per molecule.

Carborane is a cluster compound composed of boron, carbon, and hydrogen atoms, and is basically a large boron compound in which hydrogenated boron fragments are connected to each other to form a boron cage.

In the present invention, azide (N₃) was conjugated to a [¹⁰B]carborane compound having 10 ¹⁰B per mole so that the compound could be conjugated to ADIBO, which is a click chemistry functional group of the albumin, thereby synthesizing [¹⁰B]carborane-N₃, the structure of which is as shown in Formula 1 below.

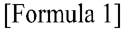

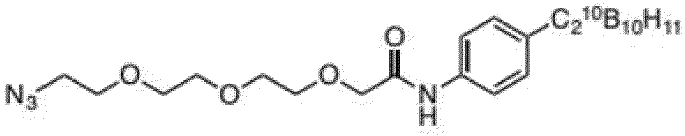

The albumin-based nanoplatform for boron neutron capture therapy of the present invention may comprise 1 to 15 boron compounds, preferably 2 to 12, and more preferably 4 to 9.

The nanoplatform of the present invention may also comprise a targeting molecule in order to accurately deliver boron to cancer cells.

As used herein, the term "targeting molecule" refers to a molecule that targets a specific environment in vivo, and specifically refers to a molecule that targets a substance present in a specific environment in vivo to guide the albumin nanoplatform of the present invention to the corresponding specific environment. The targeting molecule of the present invention may guide the albumin nanoplatform of the present invention to a specific environment, or may confirm the presence or absence of a specific environment, the location of a specific environment, the degree of a specific environment, and the like. In the present invention, the specific environment in vivo targeted by the targeting molecule may be a tissue-specific specific environment or a disease-specific specific environment. That is, the targeting molecule may target a substance that is present in a tissue-specific manner or a substance that is present in a disease-specific manner. The substance present in a tissue-specific manner may be a protein, RNA, an accumulated or generated compound, or the like that is present in a tissue-specific manner, and the substance present in a disease-specific manner may be a protein, RNA, an accumulated or generated compound, or the like that is present depending on the type of disease.

The albumin-based nanoplatform of the present invention may comprise 1 to 10 of the targeting molecules, and more preferably 1 to 8. In this case, upon injection into the human body, the targeting possibility of the nanoplatform to the target site can be increased, thereby increasing the possibility of uptake at the target site.

In particular, in the present invention, the targeting molecule may be folate or a derivative thereof, a saccharide compound, or RGD (arginyl-glycyl-aspartic acid) or a derivative thereof, which targets a folate receptor that is abundantly present in cancer tissues.

In a preferred embodiment of the present invention, the targeting molecule comprises folate.

Folate is a type of vitamin, and is also called vitamin B9 or vitamin M. Folate forms a specific binding with a folate receptor. The folate receptor is a tumor-associated glycosylphosphatidylinositol anchor protein, and allows bound folate or a folate-bound substance to be taken up through receptor-mediated endocytosis.

In the present invention, it was confirmed that the albumin nanoplatform conjugated with folate can accurately deliver a boron compound to cancer cells by targeting folate receptors overexpressed in head and neck cancer, ovarian cancer, lung cancer, cervical cancer, and the like.

The folate may be conjugated to the albumin nanoplatform in an amount of 1 to 10, and preferably 1 to 6.

In the present invention, the targeting molecule may comprise a saccharide compound. The saccharide compound may be a mannosyl group, a galactosyl group, or a glucosyl group. The structures of the mannosyl group, the galactosyl group, and the glucosyl group are as shown in Formulas 2 to 4 below, respectively.

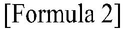

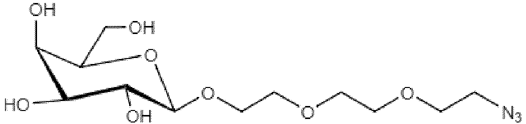

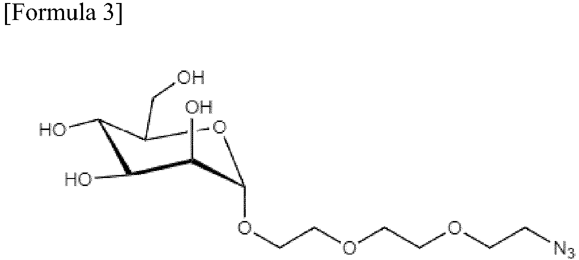

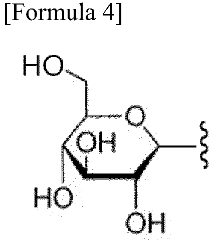

Specifically, an albumin nanoplatform comprising a mannosyl group or a galactosyl group as a transmitter has targeting ability toward macrophages, whereby drug delivery to macrophages becomes possible, and as a result, it can function as a platform for the preemptive diagnosis and treatment of metastatic cancer.

The mannose receptor is known to be widely distributed in cells responsible for defense mechanisms, among which representative immune cells are Kupffer cells. When the transmitter is a mannosyl group, since it targets Kupffer cells, it mostly acts in the liver, but its action may also occur in the blood, muscle, spleen, and lung.

In addition, when the transmitter is a galactosyl group, it may exhibit hepatobiliary excretion through the gall bladder. Furthermore, the albumin-based nanoplatform of the present invention conjugated with a glucosyl group as a transmitter has targeting ability toward M1-type macrophages, whereby it is selectively taken up by inflammatory macrophages in damaged tissues, suppressing the inflammatory response and ROS generation induced by inflammatory macrophages, thereby restoring the function of damaged mitochondria and suppressing cell death in the damaged tissues, so that it can be used as a nanoplatform capable of preventing or treating progression to a serious disease.

In the present invention, the albumin nanoplatform may comprise 4 to 8 saccharide compounds, and more preferably 5 to 7.

In addition, in the present invention, the targeting molecule may comprise RGD.

RGD is a tripeptide composed of three amino acids, that is, arginine (L-arginine), glycine, and aspartic acid (L-aspartic acid), and was first discovered at the cell adhesion site of fibronectin. Since RGD has a strong affinity for integrin receptors, which are mainly expressed in the endothelial cell layer of tumor blood vessels, it is widely used as a universal means in research such as drug-targeted therapy and simple reagents.

The nanoplatform of the present invention may further comprise, in addition to the boron compound and the targeting molecule, one or more transmitters, and in this case, a plurality of substances may be subjected to a click chemistry reaction with the albumin conjugated with the azide or cyclooctyne functional group at the same time, or a plurality of substances may be sequentially subjected to a click chemistry reaction, respectively.

In the present invention, the transmitter may further comprise a radioactive isotope, and the radioactive isotope may be labeled by a chelating agent.

A radioactive isotope refers to an element having the same atomic number but a different atomic weight, and an isotope having radioactivity is referred to as a radioactive isotope. Such a radioactive isotope can be used as an important marker for diagnosing a disease or confirming pharmacokinetics by utilizing the property of attenuating radioactivity by emitting gamma rays or other subatomic particles. The radioactive isotope that can be used as a marker in the present invention may be used without limitation as long as it is known in the art, and may be at least one selected from the group consisting of ³H, ¹¹C, ¹⁸F, ¹⁴Cl, ³²P, ³⁵S, ³⁶Cl, ⁴⁵Ca, ⁵¹Cr, ⁵⁷Co, ⁵⁸Co, ⁵⁹F, ⁶⁴Cu, ⁶⁷Ga, ⁶⁸Ga, ⁸⁹Zr, ⁹⁰Y, ⁹⁹Mo, ^{99m}Tc, ¹¹¹In, ¹³¹I, ¹²⁵I, ¹²⁴I, ¹²³I, ¹⁸⁶Re, ¹⁸⁸Re, ²²⁵Ac, ²¹²Pb, ^{117m}Sn, and ¹⁷⁷Lu, and preferably ¹¹C, ¹⁸F, ⁶⁴Cu, ⁶⁷Ga, ⁶⁸Ga, ⁸⁹Zr, ^{99m}Tc, ¹¹¹In, ¹²³I, or the like, but is not necessarily limited thereto.

The chelating agent serves to connect the radioactive isotope to the albumin, and may be, for example, but is not necessarily limited to, at least one selected from the group consisting of NOTA (1,4,7-triazacyclononane-1,4,7-triacetic acid), DOTA (1,4,7,10-tetraazacyclododecane-1,4,7,10-tetraacetic acid), DFO (3-[6,17-dihydroxy-7,10,18,21-tetraoxo-27-[N-acetylhydroxylamino]-6,11,17,22-tetraazaheptaeicosane]thiourea), DTPA (diethylenetriaminepentaacetic acid), N₂S₂ (diaminedithiol), p-SCN-Bn-NOTA (2-(4'-isothiocyanatobenzyl)-1,4,7-triazacyclononane-1,4,7-triacetic acid), NODAGA (1,4,7-triazacyclononane-1-glutaric acid-4,7-acetic acid), p-SCN-Bn-DOTA (2-(4'-isothiocyanatobenzyl)-1,4,7,10-tetraazacyclododecane-1,4,7,10-tetraacetic acid), TETA (1,4,8,11-tetraazacyclotetradecane-1,4,8,11-tetraacetic acid), p-SCN-Bn-DTPA (2-(4-isothiocyanatobenzyl)-diethylenetriaminepentaacetic acid), p-SCN-Bn-DFO (1-(4-isothiocyanatophenyl)-3-[6,17-dihydroxy-7,10,18,21-tetraoxo-27-[N-acetylhydroxylamino]-6,11,17,22-tetraazaheptaeicosane]thiourea), and HYNIC (hydrazinonicotinic acid).

The transmitter can be labeled with a radioactive isotope by comprising the radioactive isotope, and the substance thus labeled with the radioactive isotope can be utilized in research for identifying the correlation between a quantitative evaluation result based on nuclear medicine imaging and a Mass result of the substance labeled with the radioactive isotope.

In an embodiment of the present invention, an albumin nanoplatform comprising a boron compound was labeled with ⁶⁴Cu, which is a radioactive isotope, to obtain a PET image, and by measuring the PET signal, it was confirmed that the boron concentration and the PET signal have a correlation.

In addition, unlike BPA, which is conventionally used, the uptake amount of the drug can be predicted in advance through quantitative result analysis based on the isotope, and it can be evaluated at which point in time neutron beam irradiation enables the most effective treatment in relation to disease treatment.

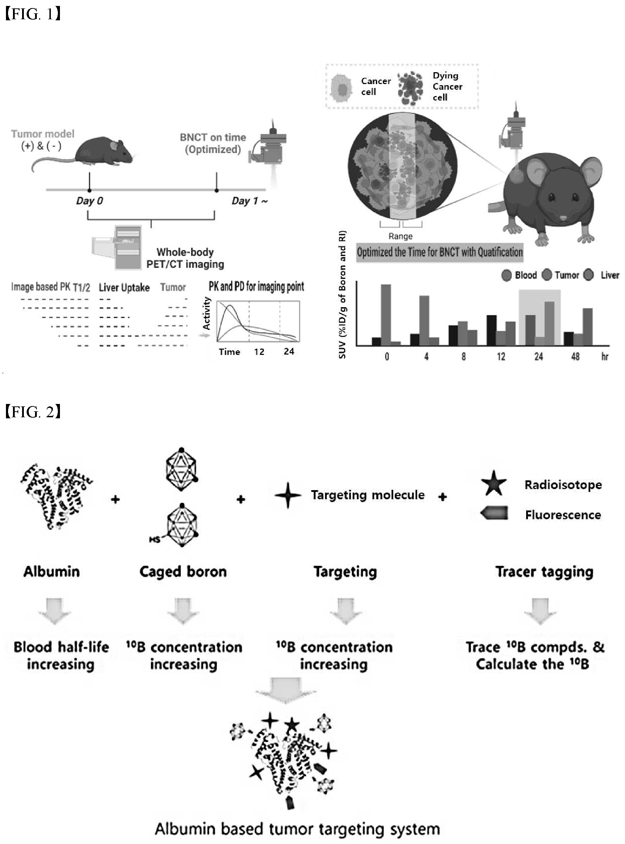

In this regard, as shown in FIG. 1, after constructing a head and neck cancer mouse model, the drug uptake in the tumor, blood, and liver is compared based on nuclear medicine imaging, and the pharmacokinetic properties related to the absorption, distribution, and excretion of the drug in the body are identified through confirmation of the residual amount of the drug in the blood, so that it can be evaluated at which point in time after actually injecting the drug the neutron beam should be irradiated to enable the most effective treatment of cancer.

A fluorescent material refers to a material that emits a specific wavelength of visible light in response to a specific wavelength, and in the present invention, all fluorescent materials that can be used to confirm the location of the nanoplatform by binding to the albumin-based nanoplatform for boron neutron capture therapy are included.

Specifically, the fluorescent material that can be used as a marker in the present invention may be used without limitation as long as it is known in the art, and fluorescent materials such as a rhodamine-based material including rhodamine, TAMRA, and the like; a fluorescein-based material including fluorescein, FITC (fluorescein isothiocyanate), and FAM (fluoresceinamidite), and the like; a bodipy-based material (bodipy, borondipyrromethene); an alexa fluor-based material; and a cyanine-based material including Cy3, Cy5, Cy7, and indocyanine green may be used, but the fluorescent material is not limited thereto.

In the present invention, the transmitter may further comprise a fluorescent material.

A fluorescent material refers to a material that emits a specific wavelength of visible light in response to a specific wavelength, and in the present invention, all fluorescent materials that can be used to confirm the location of the nanoplatform by binding to the albumin-based nanoplatform for boron neutron capture therapy are included.

Specifically, the fluorescent material that can be used as a marker in the present invention may be used without limitation as long as it is known in the art, and fluorescent materials such as a rhodamine-based material including rhodamine, TAMRA, and the like; a fluorescein-based material including fluorescein, FITC (fluorescein isothiocyanate), and FAM (fluoresceinamidite), and the like; a bodipy-based material (bodipy, borondipyrromethene); an alexa fluor-based material; and a cyanine-based material including Cy3, Cy5, Cy7, and indocyanine green may be used, but the fluorescent material is not limited thereto.

In the present invention, the albumin-based nanoplatform for boron neutron capture can accurately deliver a boron compound to a target cancer cell by selectively targeting a cancer cell or cancer tissue in which a folate receptor is overexpressed.

Another aspect of the present invention provides a composition for boron neutron capture therapy comprising the albumin-based nanoplatform.

According to an embodiment of the present invention, an albumin nanoplatform (Alb-ADIBO¹¹-[¹⁰B]⁶-FA⁵-FNR648 or Alb-ADIBO¹¹-FA³-[¹⁰B]⁶-FNR648) obtained by conjugating [¹⁰B]carborane, which is a boron compound, and folate, which is a targeting molecule, through a click chemistry reaction, as shown in FIG. 2, was injected into a cancer mouse model constructed by transplanting a head and neck cancer cell line, and in vivo fluorescence expression was confirmed; as a result of excising and quantifying cancer tissues from each experimental animal, it was confirmed that the fluorescence expression was increased by about 60% in the cancer tissue in which the folate receptor was overexpressed compared to the cancer tissue in which the folate receptor was underexpressed. Accordingly, it was confirmed that the albumin nanoplatform of the present invention can selectively target a cancer tissue, thereby effectively delivering boron to the cancer tissue.

In the case of BPA, which is a conventional BNCT drug, it has been reported that the accumulation concentration of ¹⁰B in cancer tissue for confirming the BNCT therapeutic effect is about 4 to 6 µg (0.4 to 0.6 µmol) per gram of cancer tissue, and that approximately 250 mg/kg of BPA is required to secure the cancer tissue concentration of the above level. In addition, in a recent animal experiment using BPA, it was reported that when 5 mg per mouse was injected, 30 µg/g was taken up in the cancer tissue, which represents a delivery capability of about 1.2% to the cancer per mouse when the cancer weight is 2 g (60 µg/5000 µg).

However, according to an embodiment of the present invention, when [¹⁰B]carborane conjugated to 16 µg of albumin per cancer mouse constructed by transplanting the head and neck cancer cell line was injected, it was confirmed that 1 to 1.5 µg/g was taken up in the cancer tissue. At this time, the weight of the cancer isolated from the mouse was 1.3 g, and based on this, when the efficiency of the albumin nanoplatform delivered to the cancer per mouse was evaluated, the delivery efficiency relative to the injected amount was confirmed to be 8 to 12%.

In addition, as a result of injecting [¹⁰B]carborane conjugated to 2.3 µg of albumin per cancer mouse constructed by transplanting the head and neck cancer cell line and confirming the uptake amount through PET imaging, it was confirmed that about 290 ng was taken up in the cancer tissue per mouse, and based on this, the delivery efficiency of the albumin nanoplatform was confirmed to be about 12.6%.

This means that the albumin nanoplatform of the present invention can effectively deliver boron molecules for BNCT treatment by selectively targeting a cancer tissue even with a dose that is more than 10 times smaller than that of BPA, which is a conventional BNCT drug.

In addition, conventional compositions for boron neutron capture therapy had a problem in that they had to be injected for a long time because they were excreted through the kidney or liver, but the composition for boron neutron capture therapy according to the present invention still remains in cancer cells even after 24 hours, so that a sufficient boron concentration can be maintained for the necessary time even with a single injection, thereby causing a change in the cancer treatment mechanism.

Still another aspect of the present invention provides a pharmaceutical composition for preventing or treating a cancer disease, comprising the albumin-based nanoplatform for boron neutron capture therapy as an active ingredient.

The cancer disease may comprise a cancer cell or cancer tissue in which a folate receptor is overexpressed, and may be, for example, at least one cancer disease selected from the group consisting of head and neck cancer, ovarian cancer, lung cancer, cervical cancer, bone cancer, breast cancer, central or peripheral nervous system cancer, gastrointestinal cancer, germ cell cancer, adenocarcinoma, blood cancer, renal-urinary tract cancer, liver cancer, pleural cancer, prostate cancer, sarcoma, and skin cancer.

The pharmaceutical composition may further comprise a suitable pharmaceutically acceptable carrier, excipient, or diluent according to a conventional method. The pharmaceutically acceptable carrier is one that is commonly used in formulation, and includes, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil.

The pharmaceutical composition of the present invention may further comprise, in addition to the above components, a lubricant, a wetting agent, a sweetening agent, a flavoring agent, an emulsifying agent, a suspending agent, a preservative, and the like. Suitable pharmaceutically acceptable carriers and formulations can be preferably formulated according to each component using the methods disclosed in Remington's Pharmaceutical Sciences (19th ed., 1995).

The pharmaceutical composition of the present invention can be administered either orally or parenterally, and parenteral administration includes intravenous injection, subcutaneous injection, intramuscular injection, intraperitoneal injection, transdermal administration, and the like.

Examples of formulations for oral administration include tablets, pills, hard and soft capsules, solutions, suspensions, emulsifying agents, syrups, granules, and the like, and these formulations may further comprise, in addition to the active ingredient, a diluent (e.g., lactose, dextrose, sucrose, mannitol, sorbitol, cellulose, and/or glycine) and a lubricant (e.g., silica, talc, stearic acid and its magnesium or calcium salt, and/or polyethylene glycol). In addition, the tablets may contain a binder such as magnesium aluminum silicate, starch paste, gelatin, tragacanth, methylcellulose, sodium carboxymethylcellulose, and/or polyvinylpyrrolidine, and may optionally contain a disintegrant or an effervescent mixture such as starch, agar, alginic acid, or its sodium salt, and/or an absorbent, a colorant, a flavoring agent, and a sweetening agent. The formulations may be prepared by a conventional mixing, granulating, or coating method.

In addition, a representative example of the formulation for parenteral administration is an injectable preparation, and examples of the solvent for the injectable preparation include water, Ringer's solution, isotonic physiological saline, and a suspension.

The sterile fixed oil of the injectable preparation may be used as a solvent or a suspending medium, and any non-irritating fixed oil including mono- and di-glycerides may be used for this purpose. In addition, a fatty acid such as oleic acid may be used for the injectable preparation.

The composition according to the present invention is administered in a pharmaceutically effective amount. In the present invention, the "pharmaceutically effective amount" means an amount sufficient to treat a disease at a reasonable benefit/risk ratio applicable to medical treatment, and the effective dosage level may be determined according to factors including the type and severity of the patient's disease, the activity of the drug, sensitivity to the drug, the time of administration, the route of administration and excretion rate, the duration of treatment, drugs used concurrently, and other factors well known in the medical field. The composition according to the present invention may be administered as an individual therapeutic agent or in combination with other therapeutic agents, may be administered sequentially or simultaneously with conventional therapeutic agents, and may be administered in a single dose or in multiple doses. It is important to administer an amount capable of obtaining the maximum effect with a minimum amount without side effects in consideration of all the above factors, and this can be easily determined by those skilled in the art.

Specifically, the effective amount of the composition according to the present invention may vary depending on the patient's age, sex, and body weight, and in general, 0.001 to 150 mg, preferably 0.01 to 100 mg per kg of body weight may be administered daily or every other day, or may be administered in divided doses of 1 to 3 times a day. However, since the dosage may be increased or decreased depending on the route of administration, sex, body weight, age, and the like, the dosage does not limit the scope of the present invention in any way.

### Examples

Hereinafter, the present invention will be described in more detail through Examples. However, these Examples are provided to illustrate the present invention by way of example by showing some experimental methods and configurations, and the scope of the present invention is not limited to these Examples.

### Preparation Example 1: Preparation of albumin-based nanoplatform for boron neutron capture therapy

### 1-1. Preparation of albumin having ADIBO functional group introduced thereinto

Human serum albumin (HSA) was dissolved in phosphate-buffered saline (PBS, pH 7.4) at a concentration of 40 mg/mL, and the albumin solution was dispensed at 20 mg/0.5 mL per vial. ADIBO-NHS was dissolved in DMSO (10 mg/50 µL) and added to the albumin solution to prepare 500 µL.

Albumin and ADIBO-NHS were reacted at a ratio of 1:14 at room temperature for 6 hours and purified with an Amicon Ultra-0.5 centrifugal filter tube to obtain Alb-ADIBO.

### 1-2. Preparation of carborane conjugated with azide functional group

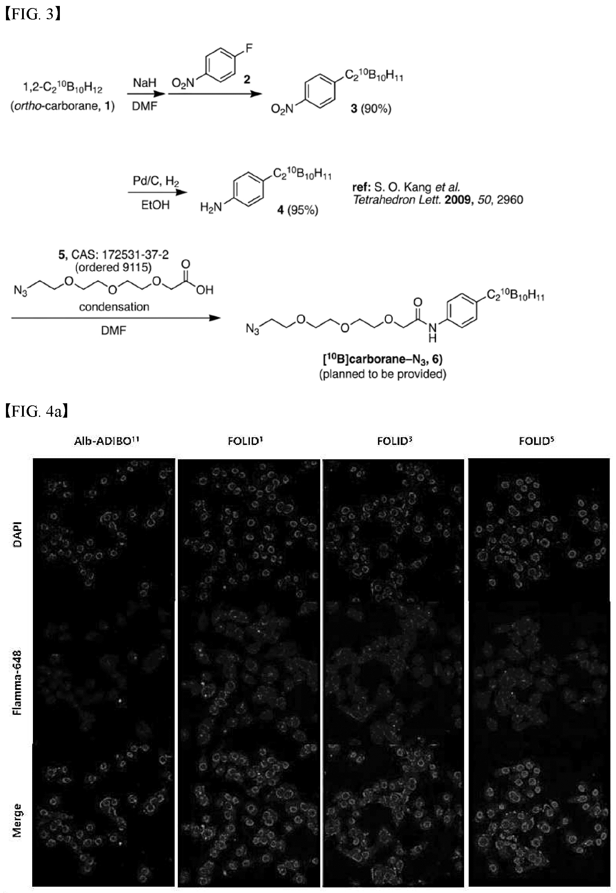

A carborane compound conjugated with ¹⁰B was prepared by the process shown in FIG. 3, and azide (N₃) capable of conjugating with the ADIBO functional group was conjugated thereto to synthesize a [¹⁰B]carborane-N₃ compound.

First, 4-o-carboranylnitrobenzene (3) was obtained from o-carborane (carborane, 1). 4-o-carboranylnitrobenzene was hydrogenated using Pd/C/H₂ to synthesize 4-o-carboranylaniline (4) in a yield of 95%.

Specifically, 4-fluoronitrobenzene (0.98 g, 7.0 mmol, 2) dissolved in 10 mL of dry DMF was added, through a cannula over 30 minutes at 0°C, to a stirred solution in which o-carborane (1.0 g, 7.2 mmol, 1) and NaH (0.24 g, 1.2 mmol) were dissolved in 20 mL of dry DMF. The reaction mixture was maintained at 0°C for 10 minutes and then slowly heated to room temperature. Thereafter, the mixture was further stirred for 1 hour, and 15 mL of a 10% aqueous HCl solution was added to the reaction mixture to terminate the reaction. The crude 4-o-carboranylnitrobenzene was extracted with AcOEt (20 mL × 2), and the organic layer was washed with distilled water (20 mL × 2) and brine, then dried over MgSO₄ and concentrated. Thereafter, the residue was purified by flash column chromatography (EA-n-hexane, 1:4, Rf = 0.53) to obtain 1.6 g (90%) of 4-o-carboranylnitrobenzene (3).

4-o-carboranylnitrobenzene was dissolved in EtOH (100 mL), and 10% Pd/C was added, followed by stirring under H₂ until a large amount of hydrogen was consumed. Thereafter, the mixture was filtered, the solvent was evaporated using a rotary vacuum concentrator, and the residue was purified by flash column chromatography (EA-n-hexane, 1:4, Rf = 0.34) to obtain 4-o-carboranylaniline (4) in an amount of 1.2 g at a yield of 95%.

Next, 11-azido-3,6,9-trioxaundecanoic acid was added in a DMF solvent to obtain azide-conjugated carborane (6).

### 1-3. Preparation of albumin nanoplatform conjugated with carborane and folate

First, an azide functional group was introduced into the amino acid side chain at the N-terminus of a folate derivative to prepare folate (Folate)-N₃.

Alb-ADIBO was dissolved in PBS and mixed with [¹⁰B]carborane-N₃ at a molar ratio of 1:8, followed by reaction at 37°C for 1 hour.

Next, folate-N₃ was mixed at a molar ratio of 1:6 and reacted at 37°C for 1 hour, thereby sequentially introducing carborane and folate.

In addition, the introduction order of carborane and folate was reversed to construct Alb-ADIBO¹¹-B¹⁰-FA and Alb-ADIBO¹¹-FA-B¹⁰, respectively, and the number of conjugations of each was confirmed as shown in Table 1 below through molecular weight confirmation using MALDI-TOF.

**[Table 1]**

| MALDI-TOF DOF | Albumin (Alb) | Boron (B¹⁰) | Folate (FA) |
|---|---|---|---|
| Alb-ADIBO¹¹-B¹⁰-FA | 1 | 6.59 | 5.64 |
| Alb-ADIBO¹¹-FA-B¹⁰ | 1 | 6.43 | 3.88 |

### 1-4. Preparation of albumin nanoplatform conjugated with fluorescent material

Using HSA-ADIBO, Alb-ADIBO¹¹-B¹⁰-FA-FNR648 and Alb-ADIBO¹¹-FAB¹⁰-FNR648 complexes conjugated with the fluorescence of FNR648-N₃ were prepared. FNR648-N₃ at 100 nmol/10 µl was prepared, and a fluorescent material in an amount equal to the number of moles of HSA-ADIBO was taken in a corresponding volume (µl) and added to the HSA-ADIBO solution, followed by reaction at 37°C for 60 minutes; thereafter, only the Alb-ADIBO¹¹-B¹⁰-FA-FNR648 and Alb-ADIBO¹¹-FA-B¹⁰-FNR648 complexes conjugated with FNR648-N₃ were separated and obtained through a PD-10 (desalting) column.

### 1-5. Preparation of albumin nanoplatform labeled with isotope

Using ⁶⁴Cu-NOTA-N3 in which ⁶⁴Cu was labeled on HSA-ADIBO and NOTA-N₃, ⁶⁴Cu-NOTA-Alb-ADIBO¹¹-B¹⁰-FA and ⁶⁴Cu-NOTA-Alb-ADIBO¹¹-FA-B¹⁰ complexes were prepared. ⁶⁴Cu-NOTA-N3 at 18 nmol/100 µl labeled with a specific activity was prepared, and ⁶⁴Cu-NOTA-N3 in an amount equal to the number of moles of HSA-ADIBO was taken in a corresponding volume (µl) and added to the Alb-ADIBO¹¹-B¹⁰-FA or Alb-ADIBO¹¹-FA-B¹⁰ solution, followed by reaction at 37°C for 30 minutes; thereafter, only the ⁶⁴Cu-NOTA-Alb-ADIBO¹¹-B¹⁰-FA and ⁶⁴Cu-NOTA-Alb-ADIBO¹¹-FA-B¹⁰ complexes conjugated with ⁶⁴Cu-NOTA-N3 were separated and obtained through purification.

### Experimental Example 1: Confirmation of targeting ability for head and neck cancer cell line according to number of conjugated folates

To target head and neck cancer, the targeting ability of an albumin nanoparticle for tumor cells was confirmed using a KB cell line in which a folate receptor (FR) is overexpressed.

First, FA-ANP was constructed by conjugating 1, 3, and 5 folate compounds (FA-N3), each having azide (N₃) introduced thereinto capable of conjugating with the ADIBO functional group of the albumin nanoparticle (ANP), and FA-ANP-FNR648 was prepared by additionally introducing a fluorescent material (FNR-648-N₃) having azide (N₃) introduced thereinto onto the surface of the albumin in order to confirm the cell internalization ability. This was treated to KB cells to confirm the cellular uptake level of the albumin nanoparticle conjugated with folate as a targeting material.

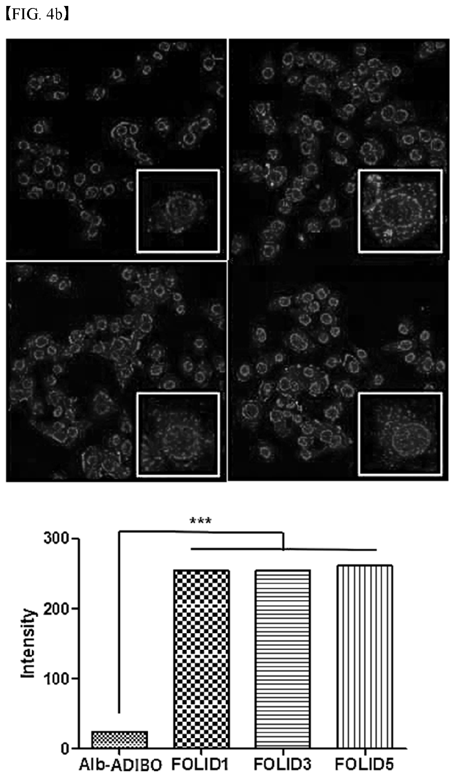

A cover glass was placed in a 12-well plate, KB cells were dispensed at 1×10⁵ cells/ml per well and cultured at 37°C for 24 hours; after replacing the medium, FA-ANP-FNR648 conjugated with 1, 3, and 5 folates, respectively, was treated, followed by culturing at 37°C under dark conditions for 1 hour. Thereafter, the cells were washed and fixed, DAPI was stained, and the fluorescence expression and expression intensity were confirmed and shown in FIGS. 4a and 4b.

As can be confirmed from FIGS. 4a and 4b, compared to the ADIBO-albumin nanoparticle not conjugated with folate, all of the albumin nanoparticles conjugated with 1, 3, and 5 folates targeted KB cells and were taken up at a very high level.

### Experimental Example 2: Confirmation of cell internalization ability of carborane-folate-albumin nanoplatform

Using a folate compound (FA-N₃) having azide (N₃) introduced thereinto capable of conjugating with the ADIBO functional group of the albumin nanoparticle (ANP) conjugated with [¹⁰B]carborane, FA-ANP was constructed, and FA-ANP-FNR648 was prepared by additionally introducing a fluorescent material (FNR-648-N₃) having azide (N₃) introduced thereinto onto the surface of the albumin in order to confirm the cell internalization ability.

Using the KB cell line of Experimental Example 1 as the experimental subject, a cell line in which the receptor was blocked by treating the cells with an excessive amount of folate in advance was prepared as a control group, and ANP-FNR648 not conjugated with folate was used as a control group and treated to the cells, after which the fluorescence expression was confirmed.

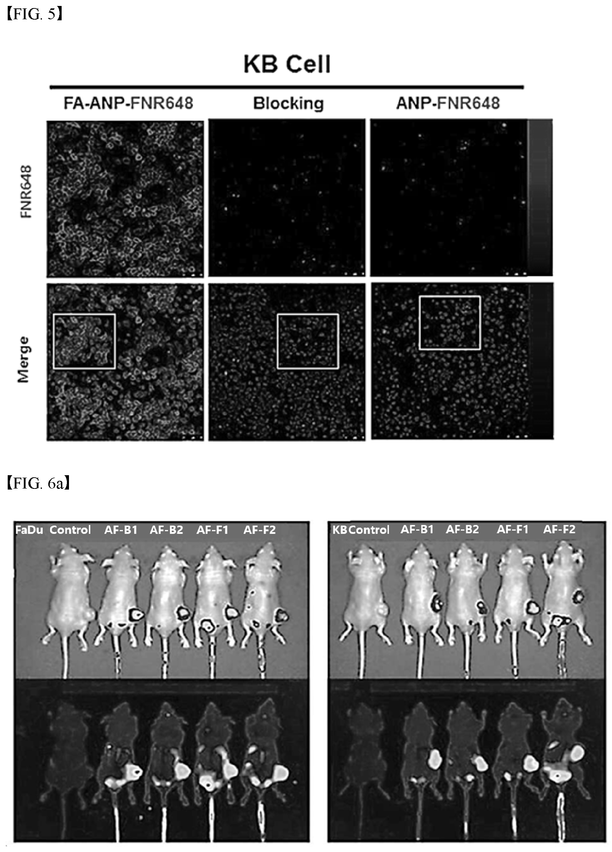

Referring to FIG. 5, it was confirmed through fluorescence that FA-ANP-FNR648 was bound to tumor cells, but in the case of the cells in which the receptor was blocked by treating an excessive amount of folate in advance and in the case of using ANP not conjugated with folate, they were not targeted to the KB cell line at all.

### Experimental Example 3: Confirmation of targeting ability for head and neck cancer animal model

Among head and neck cancer cell lines, a KB cell line in which a folate receptor is overexpressed and a FaDu cell line in which a folate receptor is underexpressed were each inoculated into nude mice to construct cancer animal models.

Using 5 animals of each animal model, 0.1 mL of Alb-ADIBO¹¹-[¹⁰B]⁶-FA⁵-FNR648 or Alb-ADIBO¹¹-FA³-[¹⁰B]⁶-FNR648 at a concentration of 0.46 mg/0.1 mL of albumin and 0.0165 mg/0.1 mL of carborane was injected, and fluorescence images were simultaneously acquired using an IVIS instrument.

In addition, fluorescence images were confirmed 24 hours after injection, and cancer tissues were excised and quantified from each animal model.

Referring to FIG. 6a, it can be confirmed that cancer tissues were selectively targeted in all animal models regardless of the introduction order of boron and folate.

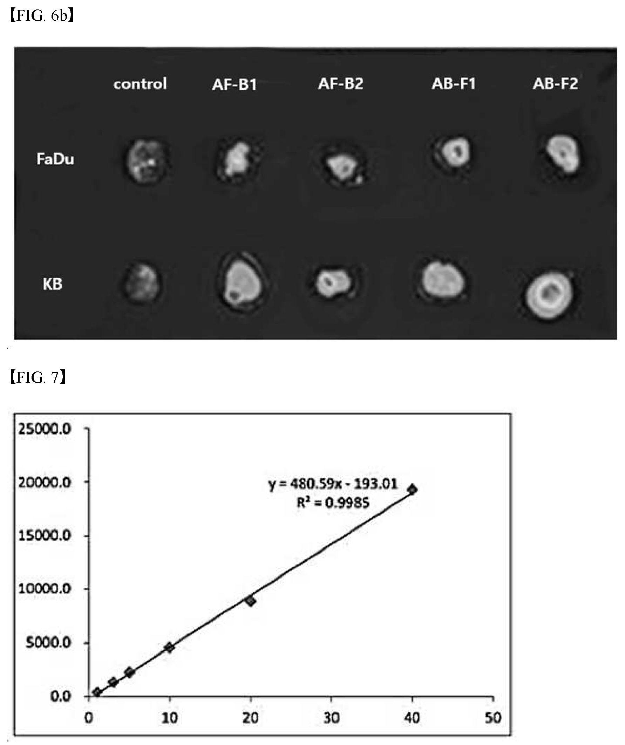

In addition, from FIG. 6b, it was confirmed that both the KB cell line and the FaDu cell line were targeted, and it was confirmed that an increase in signal of about 60% appeared in the KB receptor in which the folate receptor was overexpressed compared to the FaDu receptor in which it was underexpressed.

### Experimental Example 4: Confirmation of intratumoral boron uptake in head and neck cancer animal model

Mass analysis was performed to confirm the boron concentration in the cancer tissue of the head and neck cancer animal model to which the albumin nanoparticle of Experimental Example 3 was administered.

First, to confirm the boron used in this experiment, a standard graph of intensity with respect to the concentration of boron was obtained as shown in FIG. 7. Next, three mice targeted with the albumin nanoparticle were selected, and cancer tissues were isolated and subjected to Mass analysis.

The cancer tissue isolated from each animal was ground and diluted 10-fold, and the concentration was determined. In addition, after finally confirming the concentration in consideration of the dilution factor, it was converted into a boron uptake amount (ng/g) per cancer weight (g) in consideration of the tissue size and shown in Table 2.

**[Table 2]**

| Sample ID | Dilution factor | Measured concentration (ng/mL) | Dilution-adjusted concentration (ng/mL) | Tissue size (g) | Tissue-size-adjusted concentration (ng/g) |
|---|---|---|---|---|---|
| KB① | 10 | 0.562 | n.d | 0.610 | - |
| KB② | 10 | 1.702 | 170.200 | 1.380 | 740.000 |
| KB③ | 10 | 2.245 | 224.500 | 1.250 | 1077.600 |
| FaDu① | 10 | 0.803 | n.d | 1.040 | - |
| FaDu② | 10 | 0.637 | n.d | 1.020 | - |
| FaDu③ | 10 | 0.462 | n.d | 0.710 | - |

It was confirmed that the uptake in the cancer tissue by the KB cell line was very high compared to the uptake in the cancer tissue by the FaDu cell line, and in particular, in the case of the FaDu cell line, the uptake rate was so low that it could not be measured.

In addition, in the case of KB② among the cancer tissues used in the experiment, since only half of the isolated cancer was used, KB②, which was confirmed to be 740 ng/g, can be converted to about 1500 ng/g. Therefore, in the case of KB, an uptake amount in the cancer tissue at a level of 1000 to 1500 ng/g could be confirmed, and when converted, it represented an uptake per cancer weight of 1 to 1.5 µg/g.

### Experimental Example 5: Conversion of uptake amount through Mass analysis

The actual uptake amount was converted for the head and neck cancer mouse model to which the albumin nanoparticle of Experimental Example 3 was administered.

The albumin nanoplatform used was based on an Alb-ADIBO¹¹-FA³-[¹⁰B]⁶ compound in which 11 ADIBOs were introduced onto the surface of the albumin and 3 folates and 6 boron compounds were conjugated. The albumin nanoplatform was injected into the mouse at 0.46 mg/0.1 mL, and since the carborane concentration in the solution at this time was 0.0165 mg/0.1 mL, when the boron injected into the mouse is converted into µg, it can be regarded that 16 µg of [¹⁰B]carborane was injected per mouse.

It was confirmed that the uptake amount according to the cancer mass in the experiment was 1 to 1.5 µg/g, and considering that the cancer weight per mouse was 1.3 g, it can be converted into 1 to 1.5 µg/g × 1.3 g = 1.3 to 1.95 µg/total tumor.

In addition, the injected [¹⁰B]carborane was 16 µg per mouse, so 1 to 1.5 µg/16 µg × 100 = 8 to 12%, and it was confirmed that about 10% of the injected amount was taken up by the cancer.

BPA, which is the most widely used boron compound for BNCT at present, is known to be taken up by the cancer at 30 µg/g when 5,000 µg per mouse is injected; when the cancer weight is 2 g, 60 µg of BPA per mouse is taken up by the tumor, which represents a very low injection amount of 1.2%.

In order to compare the intratumoral uptake amount of the albumin nanoparticle of the present invention with the intratumoral uptake amount of BPA, the uptake amount of the albumin nanoparticle was converted in the same manner as the BPA.

When 16 µg of the albumin nanoparticle was injected, an uptake amount per cancer weight of 1.6 µg/g, which is about 10%, was confirmed, and in order to achieve an uptake per cancer weight of 30 µg/g like the BPA, it must be injected at a level of about 18 times.

That is, 288 µg of [¹⁰B]carborane must be injected into the mouse, and since 16 µg of [¹⁰B]carborane is conjugated per 0.46 mg of albumin, about 10 mg of albumin is required.

However, the amount of 288 µg of the boron compound is more than 50 times smaller than 5,000 µg of the BPA compound, and in the case of [¹⁰B]carborane, since there are 10 [¹⁰B] per mole, the same therapeutic effect can be exhibited even when injected at a level of 1/10, and even with only 1 mg of albumin and 28.8 µg of [¹⁰B]carborane per mouse, the same cancer uptake amount as 5,000 µg of BPA can be exhibited. When the above mouse injection dose is converted into a drug dose notation, it can be administered as a 2 mL volume injection with 50 mg/kg of the albumin nanoplatform comprising about 1.4 mg/kg of [¹⁰B]carborane.

### Experimental Example 6: Confirmation of targeting ability through nuclear medicine imaging evaluation in head and neck cancer animal model

Nuclear medicine distribution evaluation was performed in a head and neck cancer animal model constructed in the same manner as in Experimental Example 3 by labeling an albumin nanoplatform, into which FA (folate-N₃) and ¹⁰B ([¹⁰B]carborane) were introduced, with ⁶⁴Cu.

First, prepared in the same manner as the method described in Preparation Example 1-5, ⁶⁴Cu-NOTA-Alb-ADIBO¹¹-B¹⁰-FA and ⁶⁴Cu-NOTA-Alb-ADIBO¹¹-FA-B¹⁰ complexes were prepared by introducing NOTA-N₃, into which ⁶⁴Cu was introduced, through a click reaction into ¹⁰B-Albumin-FA constructed by introducing 1 to 6 FA (folate-N₃) and 1 to 9 [¹⁰B]carboranes.

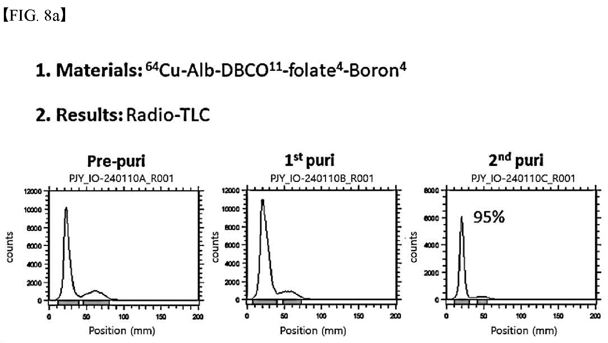

The labeling efficiency of the prepared complex was confirmed by Radio-TLC, and the result is shown in FIG. 8a; after final purification, a purity of 95% or more was confirmed and then used in animal experiments.

The same amount was injected into a total of 18 head and neck cancer mouse models, and 3 mice each were sacrificed over time to obtain blood and tumor tissues, and the size of the obtained tumor tissue and the activity of the radioactive isotope are shown in Table 3 below.

**[Table 3]**

| **0h** | | | **2h** | | |
|---|---|---|---|---|---|
| **Mouse No.** | **Tumor size (nm)** | **Activity (µCi)** | **Mouse No.** | **Tumor size (nm)** | **Activity (µCi)** |
| 3-5 | 632.27 | 64 | 3-4 | 273.26 | 61 |
| 1-3 | 142.81 | 61 | 3-1 | 215.46 | 60 |
| 1-4 | 80.62 | 64 | 2-5 | 105.45 | 60 |

| **4h** | | | **8h** | | |
|---|---|---|---|---|---|
| **Mouse No.** | **Tumor size (nm)** | **Activity (µCi)** | **Mouse No.** | **Tumor size (nm)** | **Activity (µCi)** |
| 2-1 | 293.58 | 65 | 2-2 | 306.24 | 56 |
| 4-5 | 155.45 | 54 | 1-2 | 148.62 | 55 |
| 1-5 | 101.53 | 56 | 2-3 | 91.88 | 53 |

| **24h** | | | **48h** | | |
|---|---|---|---|---|---|
| **Mouse No.** | **Tumor size (nm)** | **Activity (µCi)** | **Mouse No.** | **Tumor size (nm)** | **Activity (µCi)** |
| 4-3 | 407.42 | 53 | 4-4 | 470.05 | 52 |
| 3-2 | 138.76 | 54 | 1-1 | 123.18 | 52 |
| 4-2 | 65.4 | 52 | 3-3 | 56.09 | 53 |

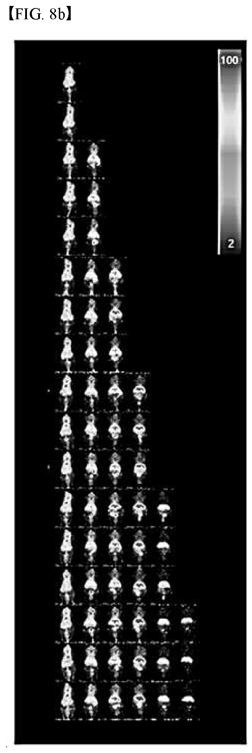

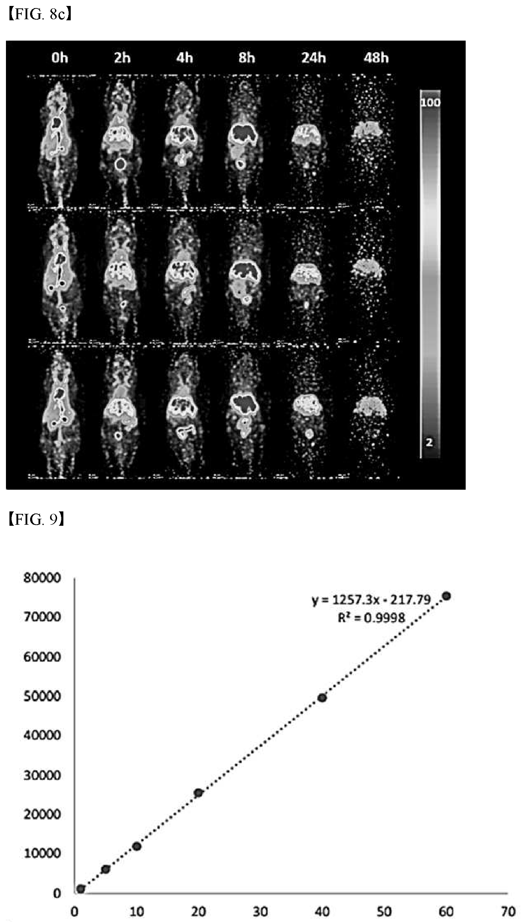

In addition, PET (Positron Emission Tomography) imaging results confirming fluorescence expression by the isotope in the 18 and final 3 head and neck cancer models are shown in FIGS. 8b and 8c, respectively.

As can be confirmed from Table 3, FIG. 8b, and FIG. 8c, it can be seen that cancer tissues were selectively targeted in all animal models, and it was confirmed that ⁶⁴Cu-labeled ¹⁰B-Albumin-FA circulated in the blood for up to 4 hours and then was gradually taken up by the liver or tumor site, remained at the tumor site even after 24 hours, and was excreted after 48 hours.

### Experimental Example 7: Confirmation and conversion of boron uptake in blood and tumor

Mass analysis was performed to confirm the boron concentration in the cancer tissue of the head and neck cancer animal model into which the albumin nanoparticle of Experimental Example 6 was injected.

To confirm the boron used in this experiment, a standard graph of intensity with respect to the concentration of boron was obtained as shown in FIG. 9. Next, Mass analysis was performed on the tumor tissues and blood isolated from the 18 mice. The tumor tissue and blood isolated from each animal were pretreated and diluted 100-fold, and the concentration was measured. In addition, the concentration was finally confirmed in consideration of the dilution factor and the tissue size, and this was converted back into ppb to obtain the final concentration, which is shown in Table 4.

**[Table 4]**

| Category | Mouse No. | Pretreatment dilution | Measured conc. (ppb) | Dilution-adjusted conc. (calc.) | Tissue size (g) | Pretreatment volume | Tissue-adjusted conc. (ng/g) | Final conc. (ppb) |
|---|---|---|---|---|---|---|---|---|
| Tumor, 0h | 1-3 | 100 | 0.333 | 33.3 | 0.116 | 2 | 574.138 | 574.138 |
| | 1-4 | 100 | 0.226 | 22.6 | 0.023 | 0.5 | 491.304 | 491.304 |
| | 3-5 | 100 | 0.554 | 55.4 | 0.275 | 2 | 402.909 | 402.909 |
| Blood, 0h | 1-3 | 100 | 2.289 | 228.9 | 0.514 | 3 | 1335.992 | 1335.992 |
| | 1-4 | 100 | 2.160 | 216 | 0.584 | 3 | 1109.589 | 1109.589 |
| | 3-5 | 100 | 2.333 | 266.6 | 0.651 | 3 | 1075.115 | 1075.115 |
| Tumor, 2h | 2-4 | 100 | 1.133 | 113.3 | 0.164 | 2 | 1381.707 | 1381.707 |
| | 2-5 | 100 | 2.226 | 222.6 | 0.091 | 0.5 | 1223.077 | 1223.077 |
| | 3-1 | 100 | 2.254 | 225.4 | 0.07 | 0.5 | 1610.000 | 1610.000 |
| Blood, 2h | 2-4 | 100 | 0.661 | 66.1 | 0.482 | 3 | 411.411 | 411.411 |
| | 2-5 | 100 | 1.194 | 119.4 | 0.769 | 3 | 465.800 | 465.800 |
| | 3-1 | 100 | 0.864 | 86.4 | 0.750 | 3 | 345.600 | 345.600 |
| Tumor, 4h | 1-5 | 100 | 1.301 | 130.1 | 0.140 | 2 | 1858.571 | 1858.571 |
| | 2-1 | 100 | 1.221 | 122.1 | 0.212 | 3 | 1727.830 | 1727.830 |
| | 4-5 | 100 | 1.170 | 117 | 0.122 | 2 | 1918.033 | 1918.033 |
| Blood, 4h | 1-5 | 100 | 1.237 | 123.7 | 0.649 | 3 | 571.803 | 571.803 |
| | 2-1 | 100 | 1.353 | 135.3 | 0.854 | 3 | 475.293 | 475.293 |
| | 4-5 | 100 | 1.026 | 102.6 | 0.639 | 3 | 481.690 | 481.690 |
| Tumor, 8h | 1-2 | 100 | 0.588 | 58.8 | 0.121 | 2 | 971.901 | 971.901 |
| | 2-2 | 100 | 0.732 | 73.2 | 0.235 | 3 | 934.468 | 934.468 |
| | 2-3 | 100 | 0.529 | 52.9 | 0.072 | 1 | 734.722 | 734.722 |
| Blood, 8h | 1-2 | 100 | 0.639 | 63.9 | 0.532 | 3 | 360.338 | 360.338 |
| | 2-2 | 100 | 0.482 | 48.2 | 0.588 | 3 | 245.918 | 245.918 |
| | 2-3 | 100 | 0.690 | 69 | 0.623 | 3 | 332.263 | 332.263 |
| Tumor, 24h | 3-2 | 100 | 0.249 | 24.9 | 0.210 | 3 | 355.714 | 355.714 |
| | 4-2 | 100 | 0.272 | 27.2 | 0.387 | 3 | 210.853 | 210.853 |
| | 4-3 | 100 | 0.215 | 21.5 | 0.300 | 3 | 215.000 | 215.000 |
| Blood, 24h | 3-2 | 100 | 0.561 | 56.1 | 0.764 | 3 | 220.288 | 220.288 |
| | 4-2 | 100 | 0.527 | 52.7 | 0.651 | 3 | 242.857 | 242.857 |
| | 4-3 | 100 | 0.401 | 40.1 | 0.657 | 3 | 183.105 | 183.105 |
| Tumor, 48h | 1-1 | 100 | 0.148 | 14.8 | 0.087 | 1 | 170.115 | 170.115 |
| | 3-3 | 100 | 0.118 | 11.8 | 0.080 | 1 | 147.500 | 147.500 |
| | 4-4 | 100 | 0.185 | 18.5 | 0.445 | 3 | 124.719 | 124.719 |
| Blood, 48h | 1-1 | 100 | 0.437 | 43.7 | 0.716 | 3 | 183.101 | 183.101 |
| | 3-3 | 100 | 0.674 | 67.4 | 0.981 | 3 | 206.116 | 206.116 |
| | 4-4 | 100 | 0.309 | 30.9 | 0.577 | 3 | 160.659 | 160.659 |

Based on the contents of Table 4, the targeting ability of the albumin nanoplatform was evaluated through conversion of the boron uptake amount for the head and neck cancer mouse model to which the albumin nanoparticle was administered.

The evaluation of the targeting ability of the nanoplatform was based on the data (tumor, 4h) for the tumor obtained from the mouse 4 hours after the albumin nanoplatform was injected.

The average of the boron concentration in consideration of the tissue size in the tumor obtained from the mouse was about 1834 ((1858 + 1727 + 1918)/3) ng/g, and multiplying the average boron concentration by the average cancer tissue size of about 0.157 ((0.140 + 0.212 + 0.122)/3) g gives about 290 ng, indicating that about 290 ng of boron per mouse is taken up by the tumor.

Since the [¹⁰B]carborane injected per mouse is about 2.3 µg, the targeting efficiency for the tumor is 0.29 µg/2.3 µg × 100% = 12.6%. This value is a result similar to the targeting efficiency of boron for the cancer tissue in Experimental Example 5, and it can be seen through this experiment as well that the albumin nanoplatform of the present invention can effectively target tumor tissue even with a very small injection amount compared to the BPA compound conventionally used.

### Experimental Example 8: Confirmation of correlation between residual boron amount in blood and nuclear medicine signal

In order to confirm the correlation between the residual boron amount in the blood and the PET image, using the blood that allowed relatively uniform sampling among the blood of the 18 mice obtained in Experimental Example 7, it was confirmed whether there was a correlation between the nuclear medicine signal (PET signal) and the residual boron amount in the blood.

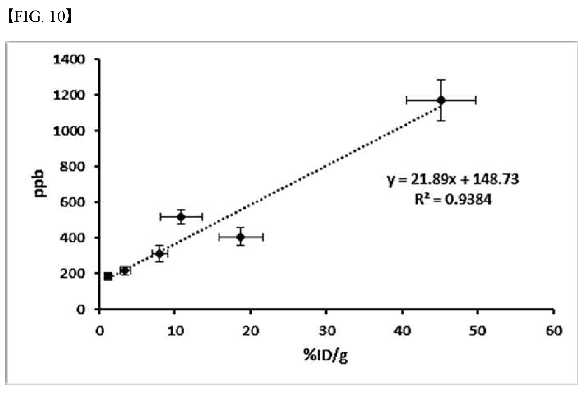

The PET signal (%ID/g), that is, the radiation signal amount and the boron concentration were measured and shown in Table 5, and based on the results of Table 5, a graph of the change in boron concentration according to the PET signal was derived and shown in FIG. 10.

**[Table 5]**

| hr | PET (%ID/g) | SD | Mass (ppb) | SD |
|---|---|---|---|---|
| 0 | 45.22 | 4.58 | 1173 | 115.39 |
| 2 | 18.75 | 2.86 | 407 | 49.07 |
| 4 | 10.84 | 2.77 | 515.67 | 39.54 |
| 8 | 7.98 | 1.02 | 312.33 | 48.96 |
| 24 | 3.41 | 0.7 | 215 | 24.34 |
| 48 | 1.22 | 0.51 | 183 | 18.78 |

As can be confirmed from the graph of FIG. 10, it can be seen that there is a correlation between the PET signal and the residual boron concentration in the blood.

Although some embodiments of the present invention have been described above, the present invention is not limited only to the embodiments described above, and various modifications and variations can be made within the scope not departing from the gist of the present invention, and it should be understood that such modified and varied forms also belong to the technical idea of the present invention.

## Claims

1. An albumin-based nanoplatform for boron neutron capture therapy (BNCT), obtained by a click chemistry reaction between albumin conjugated with an azide (N₃) or cyclooctyne functional group and a transmitter conjugated with an azide (N₃) or cyclooctyne functional group,
wherein the transmitter comprises at least one selected from the group consisting of a boron compound and a targeting molecule, and
when the albumin is conjugated with the azide functional group, the transmitter is conjugated with the cyclooctyne functional group, and when the albumin is conjugated with the cyclooctyne functional group, the transmitter is conjugated with the azide functional group.

2. The albumin-based nanoplatform for boron neutron capture therapy according to claim 1, wherein the azide or cyclooctyne functional group introduced into the albumin ranges from 1 to 30.

3. The albumin-based nanoplatform for boron neutron capture therapy according to claim 1, wherein the nanoplatform comprises 1 to 15 boron compounds.

4. The albumin-based nanoplatform for boron neutron capture therapy according to claim 1, wherein the boron compound is at least one selected from the group consisting of carborane and sodium borocaptate (BSH).

5. The albumin-based nanoplatform for boron neutron capture therapy according to claim 1, wherein the nanoplatform comprises 1 to 10 targeting molecules.

6. The albumin-based nanoplatform for boron neutron capture therapy according to claim 1, wherein the targeting molecule comprises at least one selected from the group consisting of folate or a derivative thereof, a saccharide compound, and RGD (arginyl-glycyl-aspartic acid) or a derivative thereof.

7. The albumin-based nanoplatform for boron neutron capture therapy according to claim 1, wherein the transmitter further comprises a radioactive isotope, and the radioactive isotope is at least one selected from the group consisting of ³H, ¹¹C, ¹⁸F, ¹⁴Cl, ³²P, ³⁵S, ³⁶Cl , ⁴⁵Ca, ⁵¹Cr, ⁵⁷Co, ⁵⁸Co, ⁵⁹F, ⁶⁴Cu, ⁶⁷Ga, ⁶⁸Ga, ⁸⁹Zr, ⁹⁰Y, ⁹⁹Mo, ^{99m}Tc, ¹¹¹In, ¹³¹I, ¹²⁵I, ¹²⁴I, ¹²³I, ¹⁸⁶Re, ¹⁸⁸Re, ²²⁵Ac, ²¹²Pb, ^{117m}Sn, and ¹⁷⁷Lu.

8. The albumin-based nanoplatform for boron neutron capture therapy according to claim 7, wherein the radioactive isotope is labeled by a chelating agent, and the chelating agent is at least one selected from the group consisting of NOTA (1,4,7-triazacyclononane-1,4,7-triacetic acid), DOTA (1,4,7,10-tetraazacyclododecane-1,4,7,10-tetraacetic acid), DFO (3-[6,17-dihydroxy-7,10,18,21-tetraoxo-27-[N-acetylhydroxylamino]-6,11,17,22-tetraazaheptaeicosane]thiourea), DTPA (diethylenetriaminepentaacetic acid), N₂S₂ (diaminedithiol), p-SCN-Bn-NOTA (2-(4'-isothiocyanatobenzyl)-1,4,7-triazacyclononane-1,4,7-triacetic acid), NODAGA (1,4,7-triazacyclononane-1-glutaric acid-4,7-acetic acid), p-SCN-Bn-DOTA (2-(4'-isothiocyanatobenzyl)-1,4,7,10-tetraazacyclododecane-1,4,7,10-tetraacetic acid), TETA (1,4,8,11-tetraazacyclotetradecane-1,4,8,11-tetraacetic acid), p-SCN-Bn-DTPA (2-(4-isothiocyanatobenzyl)-diethylenetriaminepentaacetic acid), p-SCN-Bn-DFO (1-(4-isothiocyanatophenyl)-3-[6,17-dihydroxy-7,10,18,21-tetraoxo-27-[N-acetylhydroxylamino]-6,11,17,22-tetraazaheptaeicosane]thiourea), and HYNIC (hydrazinonicotinic acid).

9. The albumin-based nanoplatform for boron neutron capture therapy according to claim 1, wherein the transmitter further comprises a fluorescent material, and the fluorescent material is at least one selected from the group consisting of FNR (Ferredoxin NADP(+) reductase), a cyanine-based fluorescent material, TAMRA (tetramethylrhodamine-5-maleimide), Flamma^{®} fluorescent material, and ICG (indocyanine green).

10. The albumin-based nanoplatform for boron neutron capture therapy according to claim 1, wherein the nanoplatform selectively targets a cancer cell or cancer tissue in which a folate receptor is overexpressed.

11. A composition for boron neutron capture therapy, comprising the albumin-based nanoplatform for boron neutron capture therapy according to any one of claims 1 to 10.

12. A pharmaceutical composition for preventing or treating a cancer disease, comprising the albumin-based nanoplatform for boron neutron capture therapy according to any one of claims 1 to 10.

13. The pharmaceutical composition according to claim 12, wherein the cancer disease comprises a cancer cell or cancer tissue in which a folate receptor is overexpressed.

14. The pharmaceutical composition according to claim 12, wherein the cancer disease is selected from head and neck cancer, ovarian cancer, lung cancer, cervical cancer, bone cancer, breast cancer, central or peripheral nervous system cancer, gastrointestinal cancer, germ cell cancer, adenocarcinoma, blood cancer, renal-urinary tract cancer, liver cancer, pleural cancer, prostate cancer, sarcoma, and skin cancer.
